# EUROPÄISCHE PATENTSCHRIFT

(11) **EP 2 919 673 B1**
(45) Veröffentlichungstag und Bekanntmachung des Hinweises auf die Patenterteilung: **30.05.2018**
(21) Anmeldenummer: 13792882.6
(22) Anmeldetag: 12.11.2013
(51) Int. Cl.: A61B 17/29, A61B 17/128, A61B 17/00

(54) **MEDIZINISCHES INSTRUMENT**
MEDICAL INSTRUMENT
INSTRUMENT MÉDICAL

(30) Priorität: 13.11.2012 AT 505092012
(43) Veröffentlichungstag der Anmeldung: 23.09.2015
(73) Patentinhaber: Klaffenböck, Johann, 5350 Strobl (AT); Klaffenböck, Lukas, 8010 Graz (AT); Mair, Julian, 81925 München (DE)
(72) Erfinder: Klaffenböck, Johann, 5350 Strobl (AT); Klaffenböck, Lukas, 8010 Graz (AT); Mair, Julian, 81925 München (DE)
(74) Vertreter: Babeluk, Michael
(86) Internationale Anmeldenummer: PCT/EP2013/073592
(87) Internationale Veröffentlichungsnummer: WO 2014/076066

(56) Entgegenhaltungen:
- WO-A1-95/27443
- AT-A4- 507 563
- US-A- 4 962 877
- US-A1- 2003 055 442
- US-A1- 2004 064 067
- US-A1- 2010 174 306
- US-A1- 2011 144 743

## Beschreibung

Die Erfindung betrifft ein medizinisches Instrument mit einem länglichen Element und einem an einem Ende des länglichen Elements anordenbaren Endeffektor, wobei der Endeffektor über einen hydraulischen Betätigungsmechanismus betätigbar ist, der hydraulische Betätigungsmechanismus einen Zylinderkörper mit zumindest einem, vorzugsweise zwei, drei, vier oder fünf Überströmkanälen für eine Hydraulikflüssigkeit, die über zumindest eine Bohrung mit dem Innenraum des Zylinderkörpers verbunden sind, aufweist, ein in dem Zylinderkörper angeordneter, entlang der Längsachse des Zylinderkörpers bewegbarer Kolben vorgesehen ist, wobei der Kolben den Innenraum des Zylinderkörpers in einen ersten Zylinderraum und in einen zweiten Zylinderraum teilt und über eine Kolbenstange mit dem Endeffektor in Verbindung steht, und zumindest ein Anschluss zur Einleitung von Hydraulikflüssigkeit in den ersten Zylinderraum sowie zumindest ein weiterer Anschluss, der mit zumindest einem Überströmkanal des Zylinderkörpers in Verbindung steht, vorgesehen sind.

In der US 4,485,817 A ist ein hydraulischer Betätigungsmechanismus für einen Endeffektor beschrieben, bei welchem ein Kolben gegen einen zweiten bewegt wird, um den für die Betätigung des Endeffektors benötigten Druck in der Hydraulikflüssigkeit aufzubauen. Nachteilig an diesem hydraulischen Betätigungsmechanismus ist insbesondere dessen komplizierter Aufbau sowie der Tatsache, dass dieser Betätigungsmechanismus die Ansteuerung von lediglich zwei definierten Betriebszustände des Endeffektors erlaubt. Insbesondere bei der Magenplikation sind jedoch häufig mehr als zwei Betriebszustände des Endeffektors notwendig, sodass der Betätigungsmechanismus für derartige Endeffektoren ungeeignet ist. Eine ähnliche Vorrichtung ist auch in der US 5,361,583 A gezeigt.

In der AT 507.653 B1 ist das oben beschriebene medizinische Instrument offenbart, wobei der hierbei beschriebene Endeffektor über zwei Greifelemente verfügt, mit welchem beispielsweise das Gewebe im Zuge einer Magenplikation fixiert wird. Andere hydraulische Betätigungssysteme sind beispielsweise in der US 4,962,877 A oder der US 2004/0064067 A1 offenbart.

Um nun sicher zu gehen, dass die beiden Greifelemente des Endeffektors an der korrekten Stelle im Gewebe eingreifen, wird üblicherweise die Position des Endeffektors am Gewebe mit Hilfe eines Gewebeeingriffselement, das beispielweise korkenzieherartig ausgebildet ist, festgelegt, bevor die beiden Greifelemente des Endeffektors zueinander bewegt werden, um die Plikation durchzuführen. Derartige Vorrichtungen gemäß dem Stand der Technik können beispielsweise der US 2003/005442 A1, der US 2011/0144743 A1 oder der US 2010/0174306 A1 entnommen werden.

In der WO 95/27443 A1 ist ein medizinisches Instrument für die Entfernung von intraluminalen Verschlüssen beschrieben, das einen Katheter mit einem Schneidkopf aufweist. Dieser Schneidkopf wird über ein hydraulisches System enlang seiner Längsachse rotierend bewegt.

Es ist nun Aufgabe der vorliegenden Erfindung, den im Stand der Technik und insbesondere in der AT 507.653 B1 beschriebenen hydraulischen Betätigungsmechanismus dahingehend weiter zu entwickeln, dass dieser den Einsatz zusätzlicher chirurgischer und/oder endoskopischer Instrumente und insbesondere deren Betätigung im Wesentlichen unabhängig von dem medizinischen Instrument, in dem sie aufgenommen sind, erlaubt.

Diese Aufgabe wird erfindungsgemäß dadurch gelöst, dass im Innenraum des Zylinderkörpers zumindest eine Durchführung für ein chirurgisches oder optisches Instrument angeordnet ist, deren Längsachse bevorzugterweise im Wesentlichen mit der Längsachse des Zylinderkörpers zusammenfällt. Gemäß der Erfindung ist hierbei die Durchführung in der Kolbenstange angeordnet. Damit kann ein zusätzliches chirurgisches Instrument oder aber auch eine optische Einheit, wie beispielsweise ein Endoskop durch das erfindungsgemäße medizinische Instrument platzsparend hindurchgeführt werden. Besonders bevorzugt ist hierbei vorgesehen, dass das chirurgische Instrument ein Gewebeeingriffselement, vorzugsweise mit korkenzieherartiger Spitze ist.

In einer besonders bevorzugten Ausführung der Erfindung weist der zumindest eine Überströmkanal einen ersten Abschnitt auf, der als Vertiefung an der Außenfläche des Zylinderkörpers im Wesentlichen normal zu der Längsachse der Zylinderkörpers verläuft und über zumindest eine Bohrung mit dem Innenraum des Zylinderkörpers in Verbindung steht. Des Weiteren weist er einen zweiten Abschnitt auf, der ebenfalls als Vertiefung an der Außenfläche des Zylinderkörpers im Wesentlichen parallel zu der Längsachse des Zylinderkörpers verläuft, sowie einen dritten Abschnitt im Boden des Zylinderkörpers, der mit einem Anschluss zum Auslass von Hydraulikflüssigkeit in Verbindung steht. Durch diese Ausführung des zumindest einen Überströmkanals wird eine besonders platzsparende Anordnung erhalten, bei welcher der Anschluss für die Zuleitung der Hydraulikflüssigkeit im Boden des Zylinderkörpers angeordnet ist, wobei der Zylinderboden in einer Ausführung der Erfindung als am Zylinderkörper anordenbare Dichtscheibe ausgeführt ist. In einer anderen Ausführung ist der Zylinderboden einstückig mit dem Zylinderkörper gefertigt. Die spezielle Ausführung des zumindest einen Überströmkanals erlaubt insbesondere eine genauere Bedienung des hydraulischen Betätigungsmechanismus und damit eine verbesserte Betätigung des Endeffektors.

Zudem ist besonders bevorzugt vorgesehen, dass die zumindest eine Bohrung des ersten Abschnitts zumindest einen Überstromkanals zwischen einem oberen und einem unteren Kolbenanschlag angeordnet ist, sodass der Kolben innerhalb des Zylinderkörpers in zumindest eine definierte Mittelstellung, die zwischen dem oberen und dem unteren Kolbenanschlag angeordnet ist, bringbar ist.

Der Einsatz des erfindungsgemäßen medizinischen Instruments ist nicht allein auf die Magenplikation beschränkt. So können unterschiedlichste Endeffektoren angebracht sein, besonders bevorzugt weist hierbei der Endeffektor zwei klauenartige Greifelemente auf, die aus einer Offenstellung in zumindest eine Schließstellung, vorzugsweise in zwei Schließstellungen zueinander bewegbar sind.

Im Folgenden wird anhand von nicht-einschränkenden Ausführungsbeispielen mit zugehörigen Figuren die Erfindung näher erläutert. Darin zeigen:
- Fig. 1: und Fig. 2 jeweils eine explodierte Darstellung des hydraulischen Betätigungsmechanismus in einer ersten Ausführung;
- Fig. 3: eine Draufsicht auf den Zylinderboden aus Fig. 1;
- Fig. 4: eine Schnittansicht des hydraulischen Betätigungsmechanismus im zusammengesetzten Zustand aus Fig. 1;
- Fig. 5: eine Schrägansicht auf den Zylinderkörper aus Fig. 1;
- Fig. 6: und Fig. 7 eine explodierte Darstellung des hydraulischen Betätigungsmechanismus in einer zweiten Ausführungsform;
- Fig. 8: eine Schrägansicht auf den Zylinderkörper aus Fig. 6;
- Fig. 9: eine Draufsicht auf den Zylinderboden aus Fig. 6;
- Fig. 10: und Fig. 11 zwei Schnittansichten des hydraulischen Betätigungsmechanismus im zusammengesetzten Zustand;
- Fig. 12: und Fig. 13 zwei Ausführungen eines Gewebeeingriffselements; und
- Fig. 14: einen Endeffektor zur Verwendung in Verbindung mit dem hydraulischen Betätigungsmechanismus.

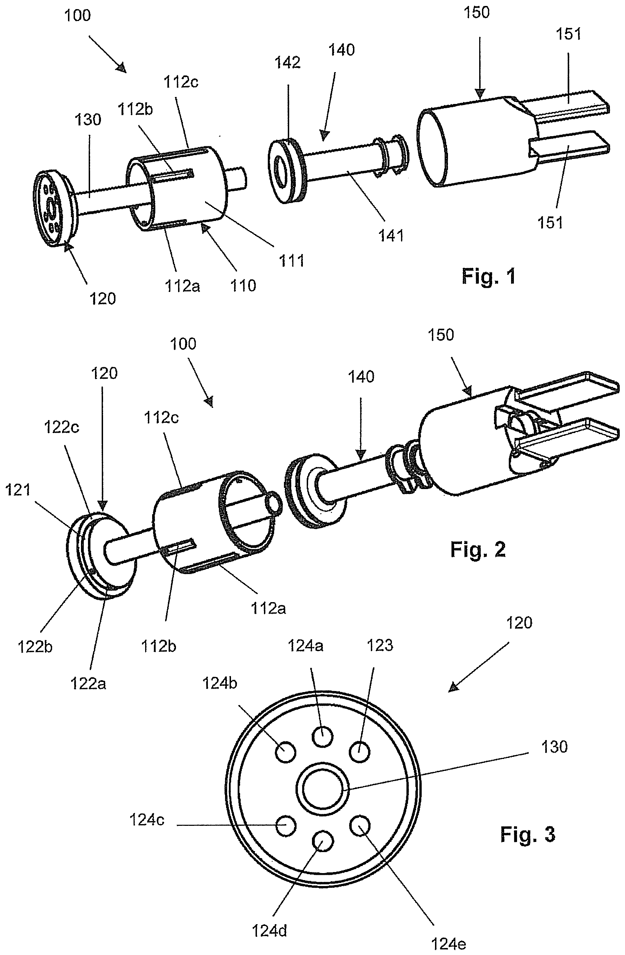

In den Figs. 1 und 2 ist jeweils eine explodierte Ansicht des hydraulischen Betätigungsmechanismus 100 für ein endochirurgisches und/oder endoskopisches Instrument gemäß der Erfindung dargestellt. Hierbei ist ein Zylinderkörper 110 vorgesehen, der als Hohlzylinder ausgeführt ist, wobei an dessen Außenwand 111 Überströmkanäle 112a, 112b, 112c angeordnet sind. Der Zylinderboden 120 wird durch eine Dichtscheibe gebildet, die dichtend an dem Zylinderkörper 110 angeordnet wird.

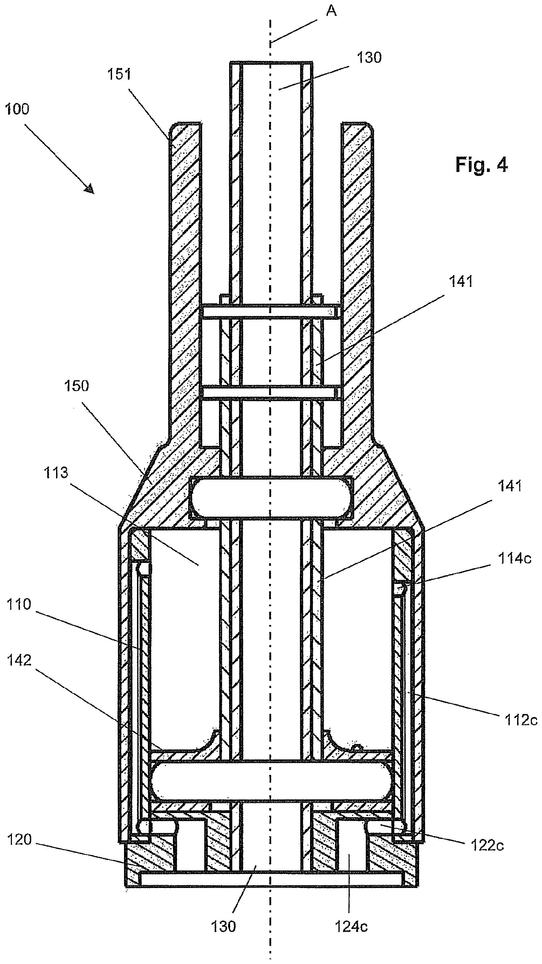

Die den Zylinderboden 120 bildende Dichtscheibe weist des Weiteren eine ringförmige Stufenkante 121 auf, an deren Flanke Durchtrittsöffnungen 122a, 122b, 122c für den Durchlass von Hydraulikflüssigkeit angeordnet sind. Diese Durchtrittsöffnungen 122a, 122b, 122c, stehen in Verbindung mit den Überströmkanälen 112a, 112b, 112c, wenn der erfindungsgemäße hydraulische Betätigungsmechanismus 100 gemäß der Fig. 4 zusammengesetzt ist.

Des Weiteren weist der hydraulische Betätigungsmechanismus 100 einen Kolben 140 auf, in dessen Kolbenstange 141 eine Durchführung 130 anordenbar ist. Im zusammengesetzten Zustand ist naturgemäß das Kolbenhemd 142 dichtend im Innenraum 113 des Zylinderkörpers 110 angeordnet und ist innerhalb des Zylinderkörpers 110 entlang dessen Längsachse A durch Einbringen von Hydraulikflüssigkeit in den Zylinderinnenraum 113 bewegbar. Die Durchführung 130 durchdringt im zusammengebauten Zustand den Zylinderboden 120 sowie den Zylinderinnenraum 113 entlang der Längsachse A des Zylinderkörper 110 (Fig. 4).

Gemäß der Fig. 3, in der die dem Zylinderinnenraum 113 abgewandte Oberfläche des Zylinderbodens 120 dargestellt ist, sind am Zylinderboden 120 eine Einlassöffnung 123 für die Einleitung von Hydraulikflüssigkeit in den Zylinderinnenraum 113 sowie Auslassöffnungen 124a, 124b, 124c, 124d, 124e, die mit den Überströmkanälen 112a, 112b, 112c in Verbindung stehen, angeordnet.

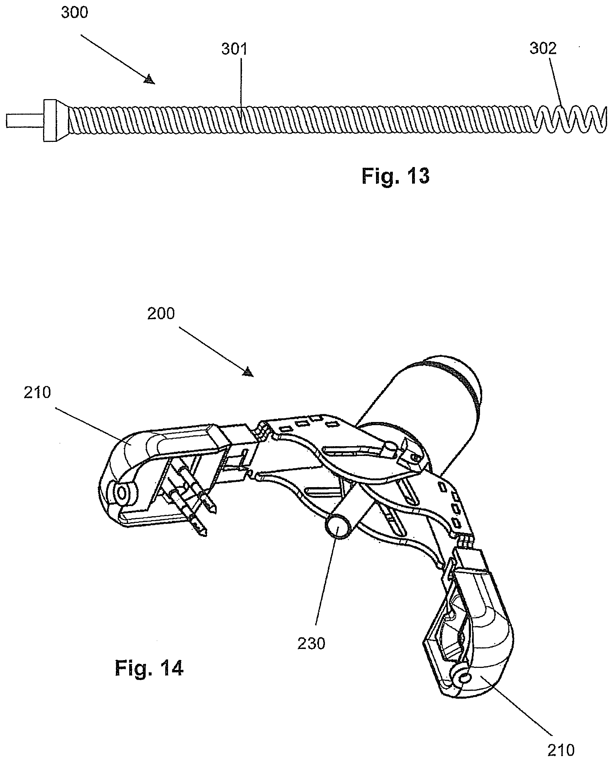

Schließlich weist der hydraulische Betätigungsmechanismus 100 eine Hülse 150 auf, die im zusammengesetzten Zustand über den Zylinderkörper 110 gestülpt ist und dichtend an dem Zylinderboden 120 angeordnet ist, sodass die Überströmkanäle 112a, 112b, 112c gegen die Umgebung abgedichtet sind. An dieser Hülse 150 sind Anschlusselemente 151 vorgesehen, an die ein Endeffektor 200 - wie beispielsweise in Fig. 14 dargestellt - anordenbar ist.

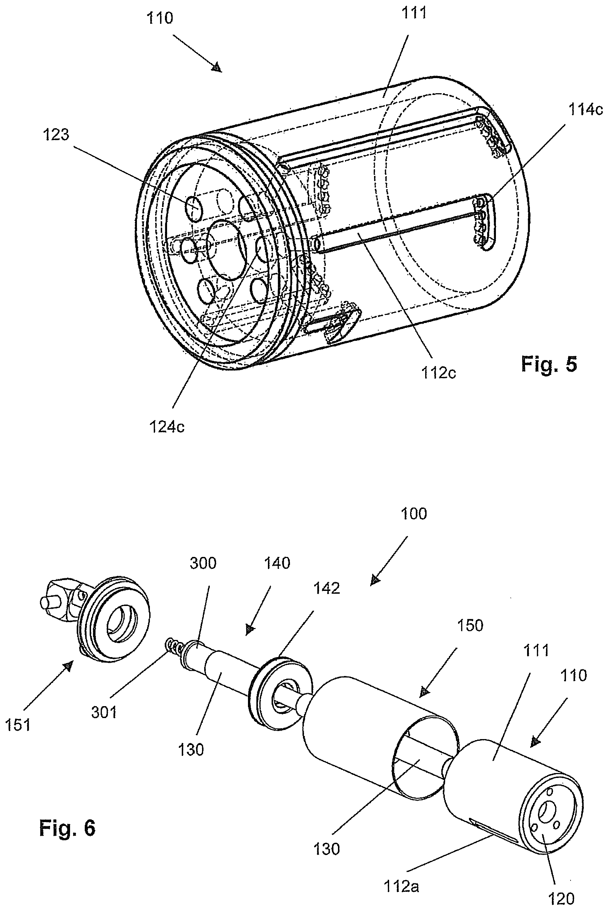

In der Fig. 4 ist der erfindungsgemäße hydraulische Betätigungsmechanismus 100 im zusammengesetzten Zustand in einer Schnittansicht gezeigt, während in der Fig. 5 der Zylinderkörper 110 in einer Schrägansicht dargestellt ist. Zur Betätigung des hydraulischen Betätigungsmechanismus 100 wird über die Einlassöffnung 123 Hydraulikflüssigkeit in den Zylinderkörper 110 eingebracht, sodass der Kolben 140 angehoben wird. Um den Kolben 140 in eine vorgegebene definierte Position zu bringen, wird eine der Auslassöffnungen 124a, 124b, 124c, 124d, 124e durch Öffnen eines Hydraulikventils freigegeben, sodass die gewünschte Kolbenhöhe, die einem definierten Betriebszustand des Endeffektors 200 entspricht, durch das Ausströmen von Hydraulikflüssigkeit aus dem Zylinderinnenraum 113 durch die Austrittsöffnung 114c erreicht wird, wobei die Hydraulikflüssigkeit anschließend durch den Überstromkanal 112c zu der Durchtrittsöffnung 122c und schließlich durch die Auslassöffnung 124c strömt.

In den Fig. 6 bis Fig. 11 ist eine weitere Ausführung des erfindungsgemäßen hydraulischen Betätigungsmechanismus 100 dargestellt.

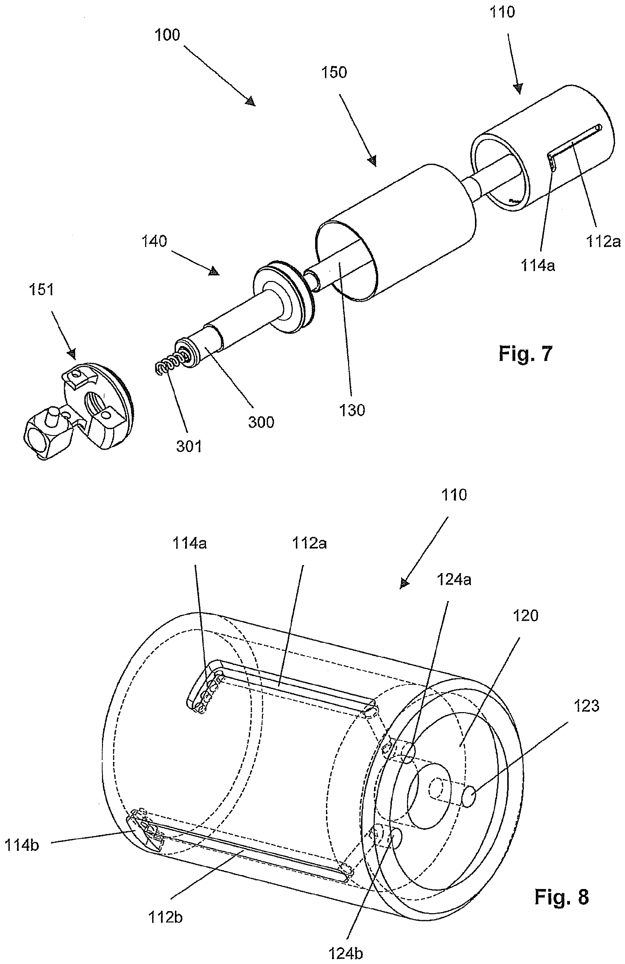

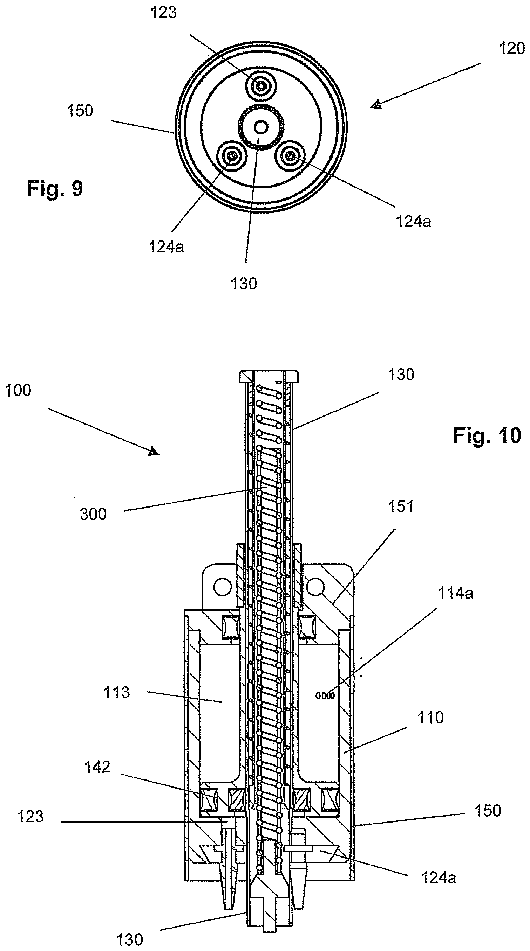

Der hydraulische Betätigungsmechanismus 100 weist wiederum einen Zylinderkörper 110 auf, der bei dieser Ausführung der Erfindung über zwei Überströmkanäle 112a, 112b mit jeweils vier Austrittsöffnungen 114a, 114b für die Hydraulikflüssigkeit verfügt. Der Zylinderboden 120 ist einstückig mit dem Zylinderkörper 110 ausgeführt, somit wird keine zusätzliche Dichtscheibe wie bei der zuvor beschriebenen Variante benötigt. Die Einlassöffnung 123 sowie die Auslassöffnungen 124a, 124b sind in dem Zylinderboden 120 angeordnet und stehen in direkter Fluidverbindung mit dem Zylinderinnenraum 113 bzw. den Überströmkanälen 112a, 112b (Fig. 8 und Fig. 9).

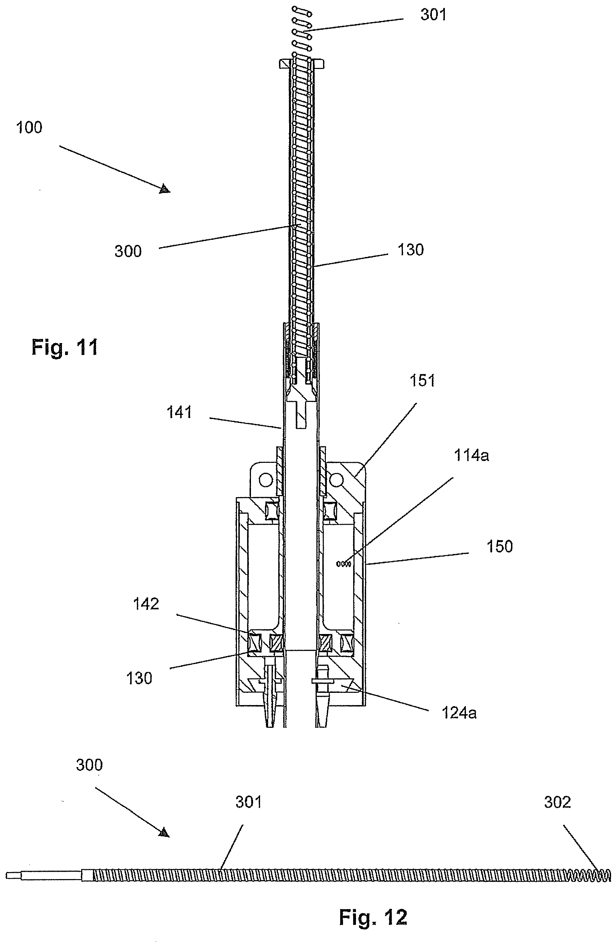

Die Kolbenstange 141 ist erneut hohl ausgeführt, wobei in ihrem Inneren wiederum eine Durchführung 130 entlang der Längsachse A des Zylinderkörpers 110 mit Hilfe eines Führungselementes, beispielsweise eines Federdrahtes (nicht dargestellt) bewegbar ist. In dieser Durchführung 130 ist in der dargestellten Ausführung der Erfindung ein Gewebeeingriffselementes 300 mit korkenzieherartiger Spitze 301 wie in Fig. 12 und Fig. 13 dargestellt zum Eingriff in Gewebe angeordnet.

Das Anschlusselement 151 für den Endeffektor 200 ist in dieser Ausführung der Erfindung als Dichtscheibe ausgebildet, durch die die Durchführung 130 hindurchgeführt wird, und die im zusammengesetzten Zustand des hydraulischen Betätigungsmechanismus 100 mit der Hülse 150 den Zylinderkörper dichtend abschließt.

In den Fig. 10 und Fig. 11 ist der hydraulische Betätigungsmechanismus 100 im zusammengesetzten Zustand jeweils in einer Schnittansicht dargestellt, wobei das in der Fig. 10 sich zur Gänze innerhalb der Durchführung 130 befindet, während es in der Fig. 11 sich in der ausgefahrenen Position befindet. Die Bewegung des Gewebeeingriffselements 300 erfolgt unabhängig der Betätigung des hydraulischen Systems und erfolgt auf an sich bekannte Weise auf mechanischem Weg.

Das Gewebeeingriffselement 300 weist in einer ersten Ausführung (Fig. 12) einen flexiblen Schaft 301 auf, an dem die korkenzieherartige Spitze 302 freistehend angeordnet ist.

Im Gebrauch in einem endochirurgischen Instrument wird dieses Gewebeeingriffselement 300 durch eine Durchführung 230 in einem Endeffektor 200 (Fig. 11) an sich bekannter Art geführt, wenn der Endeffektor 200 an dem Anschlusselement 151 des erfindungsgemäßen hydraulischen Betätigungsmechanismus 100 angeordnet ist. Damit kann das Gewebe mithilfe der korkenzieherartigen Spitze 302 des Gewebeeingriffselements 300 in Position gehalten werden, bevor die Greifelemente 210 des Endeffektors 200 um das derart fixierte Gewebe geschlossen werden, um an dem Gewebe beispielsweise eine Naht anzubringen.

Es versteht sich, dass die Erfindung nicht auf die dargestellte Ausführungsbeispiele beschränkt ist. Insbesondere kann die Anzahl und Position der Überströmkanäle unterschiedlich sein, ebenso können unterschiedliche Instrumente in der Durchführung des hydraulischen Betätigungsmechanismus angeordnet sein, sowie unterschiedlichtste Endeffektoren an den Anschlusselementen anschließbar sein.

## Patentansprüche

1. Medizinisches Instrument mit einem länglichen Element und einem an einem Ende des länglichen Elements anordenbaren Endeffektor (200), wobei der Endeffektor (200) über einen hydraulischen Betätigungsmechanismus (100) betätigbar ist, der hydraulische Betätigungsmechanismus (100) einen Zylinderkörper (110) mit zumindest einem, vorzugsweise zwei, drei, vier oder fünf Überströmkanälen (112a, 112b, 112c) für eine Hydraulikflüssigkeit, die über zumindest eine Bohrung mit dem Innenraum des Zylinderkörpers (110) verbunden sind, aufweist, ein in dem Zylinderkörper (110) angeordneter, entlang der Längsachse (A) des Zylinderkörpers (110) bewegbarer Kolben (140) vorgesehen ist, wobei der Kolben (140) den Innenraum des Zylinderkörpers (110) in einen ersten Zylinderraum und in einen zweiten Zylinderraum teilt und über eine Kolbenstange (141) mit dem Endeffektor (200) in Verbindung steht, und zumindest ein Anschluss (123) zur Einleitung von Hydraulikflüssigkeit in den ersten Zylinderraum sowie zumindest ein weiterer Anschluss (124a, 124b, 124c, 124d, 124e), der mit zumindest einem Überströmkanal (112a, 112b, 112c) des Zylinderkörpers (110) in Verbindung steht, vorgesehen sind, **dadurch gekennzeichnet, dass** im Innenraum (113) des Zylinderkörpers (110) zumindest eine Durchführung (130) für ein chirurgisches und/oder optisches und/oder therapeutisches Instrument angeordnet ist, wobei die Durchführung (130) in der Kolbenstange (141) angeordnet ist.

2. Medizinisches Instrument nach Anspruch 1, **dadurch gekennzeichnet, dass** das chirurgische Instrument ein Gewebeeingriffselement (300), vorzugsweise mit korkenzieherartiger Spitze (301), ist.

3. Medizinisches Instrument nach Anspruch 1 oder 2, **dadurch gekennzeichnet, dass** der zumindest eine Überströmkanal (112a, 112b, 112c) als Vertiefung an der Außenwand (111) des Zylinderkörpers (110) ausgebildet ist, und über zumindest eine Bohrung (114c) mit dem Innenraum (113) des Zylinderkörpers (110) in Verbindung steht, und der zumindest eine Überströmkanal (112a, 112b, 112c) mit zumindest einem Anschluss (124a, 124b, 124c, 124d, 124e) für die Hydraulikflüssigkeit in Verbindung steht.

4. Medizinisches Instrument nach einem der Ansprüche 1 bis 3, **dadurch gekennzeichnet, dass** der zumindest eine Überströmkanal (112a, 112b, 112c) einen ersten Abschnitt aufweist, der als Vertiefung an der Außenwand (111) des Zylinderkörpers (110) im Wesentlichen normal zu der Längsachse (A) des Zylinderkörpers (110) verläuft und über zumindest eine Bohrung (114c) mit dem Innenraum (113) des Zylinderkörpers (110) in Verbindung steht, der zumindest eine Überströmkanal (112a, 112b, 112c) einen zweiten Abschnitt aufweist, der als Vertiefung an der Außenfläche des Zylinderkörpers (110) im Wesentlichen parallel zu der Längsachse (A) des Zylinderkörpers (110) verläuft, und der zumindest eine Überströmkanal (112a, 112b, 112c) einen dritten Abschnitt im Boden des Zylinderkörpers (110) aufweist, der mit einem Anschluss (124a, 124b, 124c, 124d, 124e) für die Hydraulikflüssigkeit in Verbindung steht.

5. Medizinisches Instrument nach Anspruch 4, **dadurch gekennzeichnet, dass** die zumindest eine Bohrung des ersten Abschnitts des zumindest einen Überströmkanals (112a, 112b, 112c) zwischen einem oberen und einem unteren Kolbenanschlag angeordnet ist, sodass der Kolben (140) innerhalb des Zylinderkörpers (110) in zumindest eine definierte Mittelstellung, die zwischen dem oberen und dem unteren Kolbenanschlag angeordnet ist, bringbar ist.

6. Medizinisches Instrument nach einem der Ansprüche 1 bis 5, **dadurch gekennzeichnet, dass** der Endeffektor (200) zwei vorzugsweise klauenartige Greifelemente (211) aufweist, die aus einer Offenstellung in zumindest eine Schließstellung, vorzugsweise in zwei Schließstellungen zueinander bewegbar sind.

## Claims

1. A medical instrument having an elongated member and an end effector (200) disposable at one end of the elongated member, wherein the end effector (200) is operable via a hydraulic actuation mechanism (100), the hydraulic actuation mechanism (100) comprises a cylinder body (110) having at least one, preferably, two, three, four or five overflow channels (112a, 112b, 112c) for a hydraulic fluid, which are connected via at least one bore to the interior of the cylinder body (110), a piston (140) is provided which is arranged in the cylinder body (110) and is movable along the longitudinal axis (A) of the cylinder body (110), wherein the piston (140) subdivides the interior of the cylinder body (110) into a first cylinder chamber and into a second cylinder chamber and is in connection via a piston rod (141) with the end effector (200), and at least one connection (123) is provided for introducing hydraulic fluid into the first cylinder chamber and at least one further connection (124a, 124b, 124c, 124d, 124e) which communicates with at least one overflow channel (112a, 112b, 112c) of the cylinder body (110), **characterised in that** in the interior (113) of the cylinder body (110) at least one passage (130) for a surgical and/or optical and/or therapeutic instrument is arranged, wherein the passage (130) is arranged in the piston rod (141).

2. Medical instrument according to claim 1, **characterised in that** the surgical instrument is a tissue-engaging element (300), preferably with corkscrew-like tip (301).

3. Medical instrument according to claim 1 or 2, **characterised in that** the at least one overflow channel (112a, 112b, 112c) is formed as a recess on the outer wall (111) of the cylinder body (110) and is in communication via at least one bore (114c) with the interior (113) of the cylinder body (110), and the at least one overflow channel (112a, 112b, 112c) is in communication with at least one connection (124a, 124b, 124c, 124d, 124e) for the hydraulic fluid.

4. Medical instrument according to one of the claims 1 to 3, **characterised in that** the at least one overflow channel (112a, 112b, 112c) has a first portion which is formed as a recess on the outer wall (111) of the cylinder body (110) substantially normal to the longitudinal axis (A) of the cylinder body (110) and is in communication via at least one bore (114c) with the interior (113) of the cylinder body (110), the at least one overflow channel (112a, 112b, 112c) has a second portion which as a recess on the outer surface of the cylinder body (110) extends substantially parallel to the longitudinal axis (A) of the cylinder body (110), and the at least one overflow channel (112a, 112b, 112c) has a third portion in the bottom of the cylinder body (110) which is in communication with a connection (124a, 124b, 124c, 124d, 124e) for the hydraulic fluid.

5. Medical instrument according to claim 4, **characterised in that** the at least one bore of the first portion of the at least one overflow channel (112a, 112b, 112c) is arranged between an upper and a lower piston stop, so that the piston (140) within the cylinder body (110) can be brought into at least one defined center position, which is arranged between the upper and lower piston stop.

6. Medical instrument according to one of the claims 1 to 5, **characterised in that** the end effector (200) has two preferably claw-like gripping elements (211) which are movable to each other from an open position into at least one closed position, preferably in two closed positions.

## Revendications

1. Instrument médical comprenant un élément allongé et un effecteur d'extrémité (200) pouvant être monté à une extrémité de l'élément allongé, l'effecteur d'extrémité (200) pouvant être actionné par l'intermédiaire d'un mécanisme d'actionnement hydraulique (100), comprenant un corps cylindrique (110) équipé d'au moins un, de préférence de deux, de trois, de quatre ou de cinq canaux de passage (112a, 112b, 112c) d'un fluide hydraulique, qui sont reliés, par l'intermédiaire d'au moins un perçage au volume interne du corps cylindrique (110), un piston (140) monté dans le corps cylindrique (110) et mobile le long de l'axe longitudinal (A) de celui-ci étant prévu, le piston (140) subdivisant le volume interne du corps cylindrique (110) en une première chambre cylindrique et en une seconde chambre cylindrique, et étant en liaison avec l'effecteur d'extrémité (200) par l'intermédiaire d'une tige de piston (141), au moins un raccord (123) permettant d'introduire un fluide hydraulique dans la première chambre cylindrique ainsi qu'au moins un autre raccord (124a, 124b, 124c, 124d, 124e) relié à au moins un canal de passage (112a, 112b, 112c) du corps cylindrique (110) étant prévus,
**caractérisé en ce que**
dans le volume interne (113) du corps cylindrique (110) est monté au moins un passage (130) pour un instrument chirurgical et/ou optique et/ou thérapeutique, situé dans la tige de piston (141).

2. Instrument médical conforme à la revendication 1,
**caractérisé en ce qu'**
il est constitué par un élément venant en prise avec un tissu (300), de préférence équipé d'une pointe en forme de tirebouchon (301).

3. Instrument médical conforme à la revendication 1 ou 2,
**caractérisé en ce que**
le canal de passage (112a, 112b, 112c) est réalisé sous la forme d'un renfoncement situé sur la paroi externe (111) du corps cylindrique (110), et est relié par l'intermédiaire d'au moins un perçage (114c) avec le volume interne (113) du corps cylindrique (110), et le canal de passage (112a, 112b, 112c) est en liaison avec au moins un raccord (124a, 124b, 124c, 124d, 124e) pour le fluide hydraulique.

4. Instrument médial conforme à l'une des revendications 1 à 3,
**caractérisé en ce que**
le canal de passage (112a, 112b, 112c) comporte un premier segment qui s'étend sous la forme d'un renfoncement situé sur la paroi externe (111) du corps cylindrique (110) essentiellement perpendiculairement à l'axe longitudinal (A) de ce corps cylindrique (110), et est relié par l'intermédiaire d'au moins un perçage (114c) avec le volume interne (113) du corps cylindrique (110), le canal de passage (112a, 112b, 112c) comporte un second segment qui s'étend sous la forme d'un renfoncement situé sur la surface externe du corps cylindrique (110) essentiellement parallèlement à l'axe longitudinal (A) de ce corps cylindrique (110), et le canal de passage (112a, 112b, 112c) comporte un troisième segment situé au fond du corps cylindrique (110) qui est relié à un raccord (124a, 124b, 124c, 124d, 124e) pour le fluide hydraulique.

5. Instrument médical conforme à la revendication 4,
**caractérisé en ce que**
le perçage du premier segment du canal de passage (112a, 112b, 112c) est situé entre une butée supérieure et une butée inférieure du piston de sorte que le piston (140) puisse être déplacé à la partie interne du corps cylindrique (110) dans au moins une position médiane définie située entre la butée supérieure et la butée inférieure du piston.

6. Instrument médical conforme à l'une des revendications 1 à 5,
**caractérisé en ce que**
l'effecteur d'extrémité (200) comporte deux éléments de préhension (211) de préférence en forme de griffes qui peuvent être déplacés à partir d'une position d'ouverte dans au moins une position fermée, en particulier dans deux positions fermées.
